(19) Europäisches Patentamt
European Patent Office
Office européen des brevets

(11) Veröffentlichungsnummer: **0 167 019**
**A1**

(12) # EUROPÄISCHE PATENTANMELDUNG

(21) Anmeldenummer: 85107107.6

(22) Anmeldetag: 10.06.85

(51) Int. Cl.⁴: **C 07 C 103/38**
C 07 C 103/60, A 01 N 37/18

(30) Priorität: 20.06.84 DE 3422918

(43) Veröffentlichungstag der Anmeldung:
08.01.86 Patentblatt 86/2

(84) Benannte Vertragsstaaten:
AT BE CH DE FR GB IT LI NL

(71) Anmelder: BAYER AG
Konzernverwaltung RP Patentabteilung
D-5090 Leverkusen 1 Bayerwerk(DE)

(72) Erfinder: Wolf, Hilmar, Dr.
Haus Gravener Strasse 105
D-4018 Langenfeld(DE)

(72) Erfinder: Naumann, Klaus, Dr.
Richard-Wagner-Strasse 9
D-5090 Leverkusen 1(DE)

(72) Erfinder: Becker, Benedikt, Dr.
Metzkausener Strasse 14
D-4020 Mettmann(DE)

(54) **Diaryl-ethylamino-Derivate.**

(57) Es werden neue Diaryl-ethylamino-Derivate der Formel

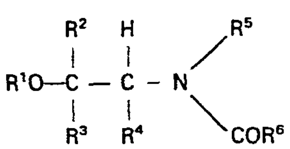

(I)

bereitgestellt, in welcher

$R^1$ für Wasserstoff, gegebenenfalls substituiertes Alkyl, Alkenyl oder Alkinyl steht,

$R^2$ und $R^3$ gleich oder verschieden sind und für substituiertes Aryl stehen,

$R^4$ für Wasserstoff oder Alkyl steht,

$R^5$ für gegebenenfalls substituiertes Alkyl, für Alkenyl, alkinyl oder Cycloalkyl steht und

$R^6$ für Wasserstoff oder Alkyl steht.

Die neuen Verbindungen können nach verschiedenen Verfahrensvarianten hergestellt werden und weisen starke biologische, insbesondere akarizide Eigenschaften auf, die ihre Verwendung als Schädlingsbekämpfungsmittel ermöglichen.

EP 0 167 019 A1

BAYER AKTIENGESELLSCHAFT 5090 Leverkusen, Bayerwerk
Konzernverwaltung RP
Patentabteilung

Er/Kü-c
Ib

## Diaryl-ethylamino-Derivate

Die vorliegende Erfindung betrifft neue Diaryl-ethylamino-Derivate, mehrere Verfahren zu ihrer Herstellung und ihre Verwendung als Schädlingsbekämpfungsmittel, insbesondere als Akarizide.

Es ist bereits bekannt, daß Diaryl-hydroxymethyl-Derivate wie z.B. 1,1-Bis-(4-chlorphenyl)-1-hydroxy-essigsäureethylester akarizide Eigenschaften besitzen (vergl. z.B. US-PS 2 745 780). Die Wirkung dieser Verbindungen ist jedoch unter bestimmten Umständen, insbesondere bei niedrigen Aufwandmengen und -konzentrationen nicht immer ganz befriedigend.

Es wurden die neuen Diaryl-ethylamino-Derivate der Formel (I)

$$R^1O-CR^2R^3-CHR^4-N\begin{matrix} R^5 \\ COR^6 \end{matrix} \qquad (I)$$

gefunden, in welcher

$R^1$ für Wasserstoff, gegebenenfalls substituiertes Alkyl, Alkenyl oder Alkinyl steht,

$R^2$ und $R^3$ gleich oder verschieden sind und für substituiertes Aryl stehen,

$R^4$ für Wasserstoff oder Alkyl steht,

$R^5$ für gegebenenfalls substituiertes Alkyl, für Alkenyl, Alkinyl oder Cycloalkyl steht und

$R^6$ für Wasserstoff oder Alkyl steht.

Weiterhin wurde gefunden, daß die neuen Diaryl-ethylamino-Derivate der Formel (I) erhalten werden, indem man

(a) Amine der Formel (II)

$$R^1O-CR^2R^3-CHR^4-NHR^5 \qquad (II)$$

in welcher

$R^1$, $R^2$, $R^3$, $R^4$ und $R^5$ die oben angegebenen Bedeutungen haben,

mit Verbindungen der Formel (III)

$$R^6-\overset{\overset{\displaystyle O}{\|}}{C}-Y \qquad (III)$$

in welcher

$R^6$ die oben angegebene Bedeutung hat und

Y für Chlor, Brom oder für den Rest der Formel

$-O-\overset{\overset{O}{\|}}{C}-R^7$ steht,

in welcher

$R^7$ für Alkyl steht,

gegebenenfalls in Gegenwart von Verdünnungsmitteln und gegebenenfalls in Gegenwart von Säureakzeptoren umsetzt,

oder

(b) Diaryl-ethylamino-Derivate der Formel (Ia)

$$HO-CR^2R^3-CHR^4-N\begin{matrix} R^5 \\ COR^6 \end{matrix} \qquad (Ia)$$

in welcher

$R^2$, $R^3$, $R^4$, $R^5$ und $R^6$ die oben angegebenen Bedeutungen haben,

mit Alkylierungsmitteln der Formel (IV)

$$R^1X \qquad (IV)$$

in welcher

$R^1$ die oben angegebene Bedeutung hat und

X für eine nucleophile Abgangsgruppe steht,

in Gegenwart von Säureakzeptoren und gegebenenfalls in Gegenwart von Verdünnungsmitteln umsetzt.

Die neuen Verbindungen der Formel (I) weisen starke biologische, insbesondere akarizide Eigenschaften auf, die ihre Verwendung als Schädlingsbekämpfungsmittel ermöglichen. Dabei zeigen die erfindungsgemäßen Verbindungen überraschenderweise eine höhere Wirkung als die bereits aus dem Stand der Technik bekannte Verbindung 1,1-Bis-(4-chlorphenyl)-1-hydroxy-essigsäureethylester.

Die Erfindung betrifft vorzugsweise Verbindungen der Formel (I), in denen

$R^1$ für Wasserstoff oder für gegebenenfalls substituiertes Alkyl mit 1 bis 6 Kohlenstoffatomen, Alkenyl oder Alkinyl mit 2 bis 6 Kohlenstoffatomen steht, wobei als Substituenten vorzugsweise genannt seien:

Halogen, Alkoxy und Alkylthio mit jeweils 1 bis 4 Kohlenstoffatomen, Halogenalkoxy und Halogenalkylthio mit jeweils 1 oder 2 Kohlenstoffatomen und 1 bis 5 gleichen oder verschiedenen Halogenatomen, wie vorzugsweise Fluor und Chloratomen; sowie Cycloalkoxy mit 2 bis 4 Kohlenstoffatomen;

$R^2$ und $R^3$ gleich oder verschieden sind und für einfach oder mehrfach, gleich oder verschieden substituiertes Aryl mit 6 bis 10 Kohlenstoffatomen stehen, wobei als Substituenten vorzugsweise in Frage kommen:

Halogen, Alkyl mit 1 bis 4 Kohlenstoffatomen, Alkoxy und Alkylthio mit 1 oder 2 Kohlenstoffatomen, Nitro, Halogenalkyl, Halogenalkoxy sowie Halogenalkylthio mit jeweils 1 oder 2 Kohlenstoffatomen und 1 bis 5 gleichen oder verschiedenen Halogenatomen, wie vorzugsweise Fluor und Chloratomen;

$R^4$ für Wasserstoff oder Alkyl mit 1 bis 4 Kohlenstoffatomen steht;

$R^5$ für gegebenenfalls substituiertes Alkyl mit 1 bis 6 Kohlenstoffatomen, Alkenyl und Alkinyl mit jeweils 2 bis 6 Kohlenstoffatomen sowie für Cycloalkyl mit 3 bis 7 Kohlenstoffatomen steht, wobei als Alkylsubstituenten vorzugsweise in Frage kommen:

0167019

Hydroxy, Alkoxy und Alkylthio mit jeweils 1 bis 4 Kohlenstoffatomen, Halogenalkoxy und Halogenalkylthio mit jeweils 1 oder 2 Kohlenstoffatomen und 1 bis 5 gleichen oder verschiedenen Halogenatomen, wie vorzugsweise Fluor und Chloratomen; und

$R^6$ für Wasserstoff oder Alkyl mit 1 bis 6 Kohlenstoffatomen steht.

Besonders bevorzugt sind diejenigen Verbindungen der Formel (I), in denen

$R^1$ für Wasserstoff, Methyl, Ethyl, Allyl, Propargyl oder Methylcyclopropoxy steht;

$R^2$ und $R^3$ gleich oder verschieden sind und für einfach oder zweifach, gleich oder verschieden substituiertes Phenyl stehen, wobei als Substituenten vorzugsweise in Frage kommen:

Fluor, Chlor, Brom, Methyl, Ethyl, n-Propyl, i-Propyl, Nitro, Methoxy, Ethoxy, Methylthio, Ethylthio, Trifluormethyl, Trifluormethoxy oder Trifluormethylthio;

$R^4$ für Wasserstoff, Methyl oder Ethyl steht;

$R^5$ für gegebenenfalls durch Hydroxy, Methoxy, Ethoxy, Methylthio oder Ethylthio substituiertes Alkyl mit 1 bis 6 Kohlenstoffatomen sowie für Allyl, Propargyl, Cyclopropyl, Cyclopentyl oder Cyclohexyl steht und

$R^6$ für Methyl, Ethyl, n-Propyl, i-Propyl, n-Butyl, i-Butyl, sec.-Butyl oder tert.-Butyl steht.

Eine ganz bevorzugte Gruppe sind diejenigen Verbindungen der Formel (I), in denen

$R^1$ für Wasserstoff, Methyl oder Ethyl steht,

$R^2$ und $R^3$ gleich oder verschieden sind und für 4-Fluorphenyl, 4-Chlorphenyl, 4-Bromphenyl und 4-Trifluormethylphenyl stehen,

$R^4$ für Wasserstoff oder Methyl steht,

$R^5$ für Methyl, Ethyl, n-Propyl, i-Propyl, Allyl und Propargyl steht und

$R^6$ für Methyl steht.

Verwendet man für das erfindungsgemäße Verfahren (a) /2,2-Bis-(4-fluorphenyl)-2-hydroxyethyl/(ethyl)amin und Acetylchlorid bzw. Essigsäureanhydrid als Ausgangsstoffe, so kann der Reaktionsablauf durch das folgende Formelschema wiedergegeben werden:

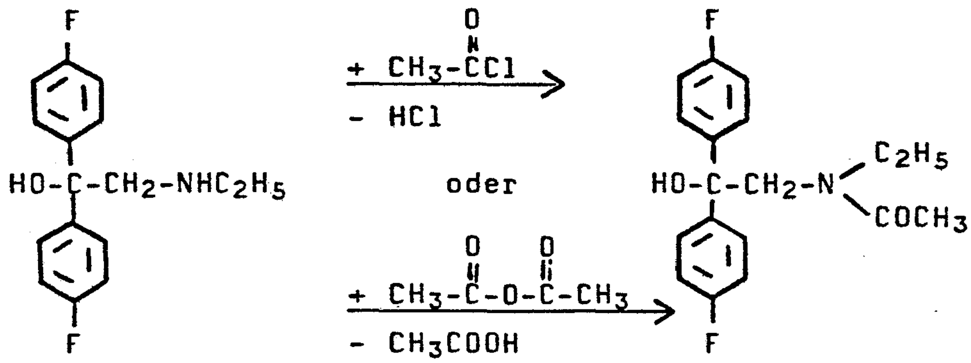


Verwendet man für das erfindungsgemäße Verfahren (b) /2,2-Bis-(4-chlorphenyl)-2-hydroxyethyl7-(methylethyl)-acetamid und Methyliodid als Ausgangsstoffe, so kann der Reaktionsablauf durch das folgende Formelschema wiedergegeben werden:

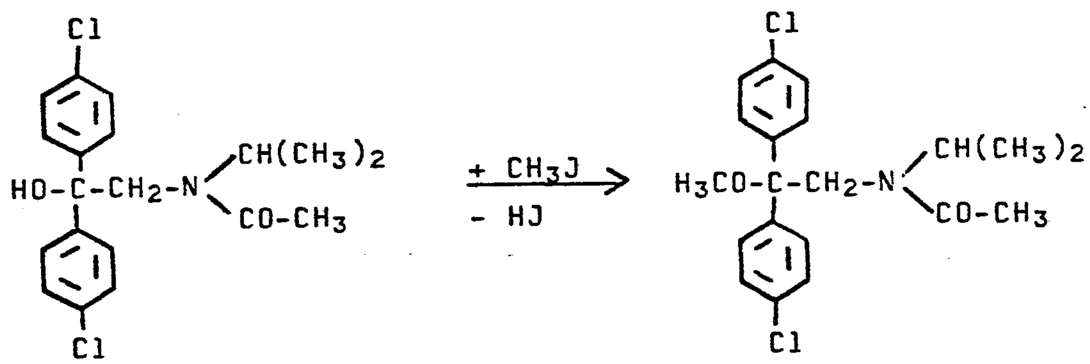


Die für das Verfahren (a) als Ausgangsstoffe zu verwendenden Amine sind durch die Formel (II) definiert. In dieser Formel haben $R^1$, $R^2$, $R^3$, $R^4$ und $R^5$, vorzugsweise diejenigen Bedeutungen, die bereits bei der Definition der Formel (I) genannt wurden.

Als Beispiele für die Ausgangsstoffe der Formel (II) seien genannt:

$$R^1O-CR^2R^3-CHR^4-NHR^5 \qquad (II)$$

Tabelle 1

| $R^1$ | $R^2$ | $R^3$ | $R^4$ | $R^5$ |
|---|---|---|---|---|
| H | 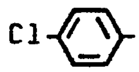 | 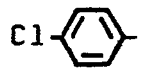 | H | $CH_3$ |
| H | 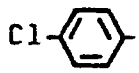 | 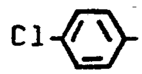 | $CH_3$ | $CH_3$ |
| $CH_3$ | 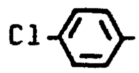 | 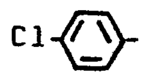 | $CH_3$ | $CH_3$ |
| H | 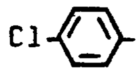 | 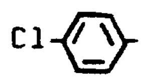 | H | $C_2H_5$ |
| H | 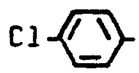 | 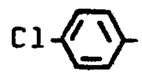 | H | i-$C_3H_7$ |
| H | 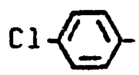 | 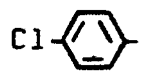 | H | i-$C_4H_9$ |
| H | 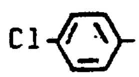 | 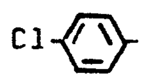 | H | 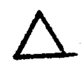 |
| H | 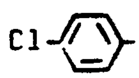 | 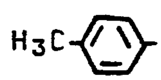 | H | i-$C_3H_7$ |
| H | 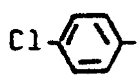 | 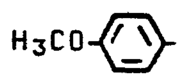 | H | i-$C_3H_7$ |
| $CH_3$ | 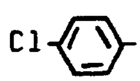 | 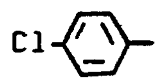 | H | i-$C_3H_7$ |
| H | 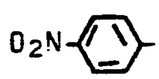 | 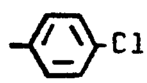 | H | i-$C_3H_7$ |
| H | 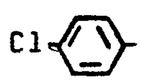 | 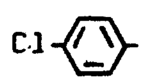 | H | sec.-$C_4H_9$ |
| H | 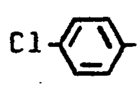 | 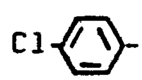 | H | 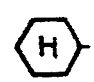 |
| H | 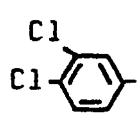 | 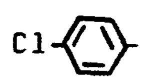 | H | i-$C_3H_7$ |

Tabelle 1 - Fortsetzung

| $R^1$ | $R^2$ | $R^3$ | $R^4$ | $R^5$ |
|---|---|---|---|---|
| H | 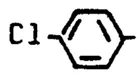 | 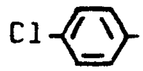 | H | $-CH(C_2H_5)_2$ |
| H | 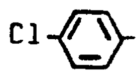 | 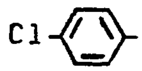 | H | $CH_3(CH_2)_2-CH-CH_3$ |
| H | 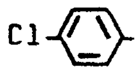 | 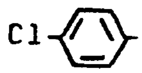 | H | $CH_3-CH-CH_2-CH(CH_3)_2$ |
| H | 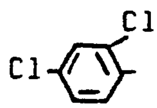 | 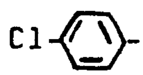 | H | $i-C_3H_7$ |
| H | 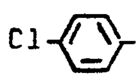 | 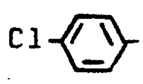 | H | $-CH_2-CH=CH_3$ |
| H | 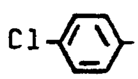 | 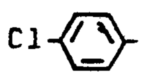 | H | $n-C_3H_7$ |
| H | 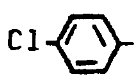 | 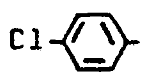 | H | $-CH_2-CH(OH)-CH_3$ |
| H | 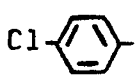 | 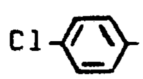 | H | $-CH_2CH_2OCH_3$ |
| H | 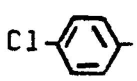 | 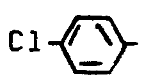 | H | $-CH_2-C\equiv CH$ |
| H | 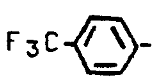 | 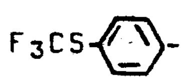 | H | $i-C_3H_7$ |
| H | 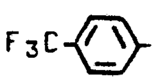 | 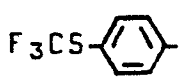 | H | $C_2H_5$ |
| H | 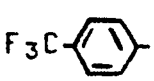 | 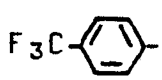 | H | $C_2H_5$ |
| H | 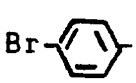 | 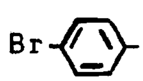 | H | $C_2H_5$ |

Tabelle 1 - Fortsetzung

| $R^1$ | $R^2$ | $R^3$ | $R^4$ | $R^5$ |
|---|---|---|---|---|
| H | 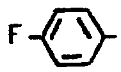 | 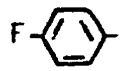 | H | $C_2H_5$ |
| H | 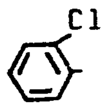 | 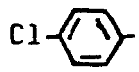 | H | $i-C_3H_7$ |
| H | 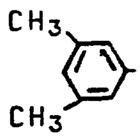 | 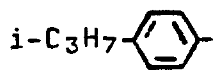 | H | $i-C_3H_7$ |
| $C_2H_5$ | 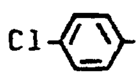 | 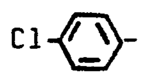 | H | $i-C_3H_7$ |
| 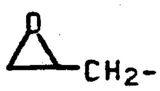 | 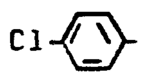 | 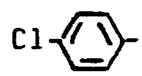 | H | $i-C_3H_7$ |
| $-CH_2-C\equiv CH$ | 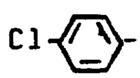 | 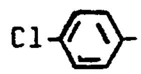 | H | $i-C_3H_7$ |
| $-CH_2-CH=CH_2$ | 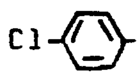 | 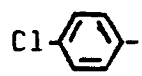 | H | $i-C_3H_7$ |
| H | 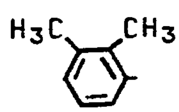 | 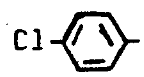 | H | $i-C_3H_7$ |
| H | 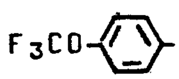 | 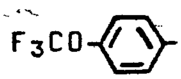 | H | $i-C_3H_7$ |
| H | 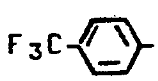 | 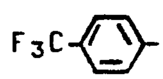 | H | $i-C_3H_7$ |
| H | 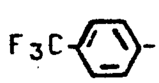 | 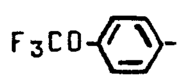 | H | $i-C_3H_7$ |
| H | 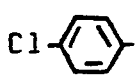 | 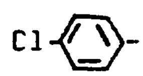 | $CH_3$ | $C_2H_5$ |

Tabelle 1 - Fortsetzung

| $R^1$ | $R^2$ | $R^3$ | $R^4$ | $R^5$ |
|---|---|---|---|---|
| H | 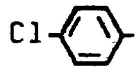 | 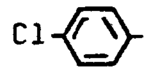 | H | $n-C_4H_9$ |
| $CH_3$ | 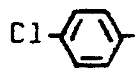 | 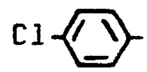 | H | $C_2H_5$ |
| $C_2H_5$ | 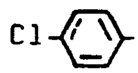 | 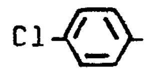 | H | $C_2H_5$ |
| H | 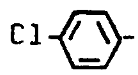 | 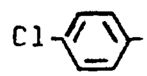 | $C_2H_5$ | $C_2H_5$ |
| H | 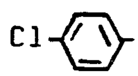 | 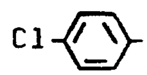 | $i-C_3H_7$ | $C_2H_5$ |
| H | 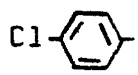 | 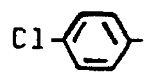 | $C_2H_5$ | $CH_3$ |
| H | 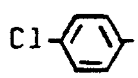 | 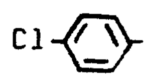 | $i-C_3H_7$ | $CH_3$ |
| H | 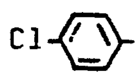 | 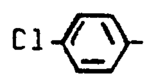 | $CH_3$ | $i-C_3H_7$ |
| H | 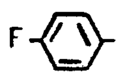 | 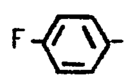 | H | $i-C_3H_7$ |
| H | 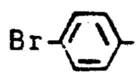 | 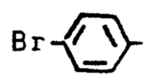 | H | $i-C_3H_7$ |
| H | 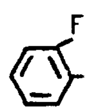 | 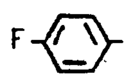 | H | $C_2H_5$ |
| H | 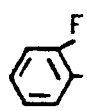 | 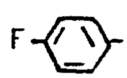 | H | $i-C_3H_7$ |
| H | 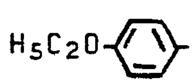 | 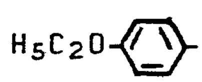 | H | $C_2H_5$ |
| H | 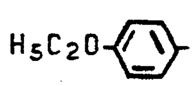 | 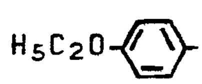 | H | $i-C_3H_7$ |

Tabelle 1 - Fortsetzung

| $R^1$ | $R^2$ | $R^3$ | $R^4$ | $R^5$ |
|---|---|---|---|---|
| $CH_3$ | 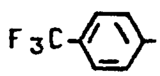 | 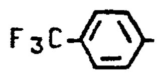 | H | $C_2H_5$ |
| $CH_3$ | 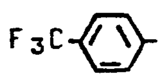 | 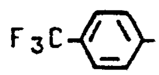 | H | $i-C_3H_7$ |
| H | 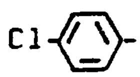 | 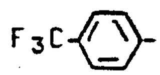 | H | $C_2H_5$ |
| H | 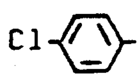 | 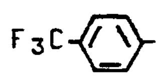 | H | $i-C_3H_7$ |
| H | 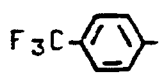 | 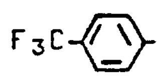 | H | $CH_3$ |
| H | 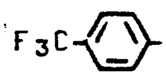 | 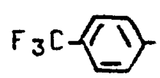 | H | $-CH_2-CH=CH_2$ |
| H | 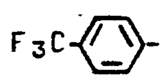 | 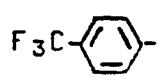 | H | $-CH_2-C\equiv CH$ |
| H | 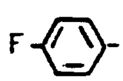 | 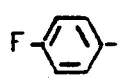 | H | $CH_3$ |
| H | 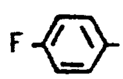 | 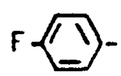 | H | $-CH_2-CH=CH_2$ |
| H | 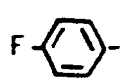 | 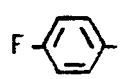 | H | $-CH_2-C\equiv CH$ |

Die Verbindungen der Formel (II) sind bekannt und/oder lassen sich auf einfacher Weise nach bekannten Methoden und Verfahren herstellen (vergl. z.B. J. Org. Chem. 35, S. 2071 (1970); CA-PS 592 332; Org. Synth. Vol. 58, S. 113 ff (1978); Chem. Pharm. Bull. 8, S. 767 ff (1960); Synth. 1979, 423; US 2 946 793).

Die für das Verfahren (a) ebenfalls als Ausgangsstoffe zu verwendenden Verbindungen sind durch die Formel (III) definiert. In dieser Formel steht $R^6$ vorzugsweise für diejenigen Reste, die bereits bei der Definition der Formel (I) genannt wurden. Y steht in dieser Formel für Chlor, Brom oder für den Rest $R^7COO-$. $R^7$ steht vorzugsweise für Methyl, Ethyl, n-Propyl, i-Propyl, n-Butyl, i-Butyl, sec.-Butyl oder tert.-Butyl.

Als Beispiele für die Ausgangsstoffe der Formel (III) seien genannt:

Essigsäureanhydrid, Acetylchlorid, Acetylbromid; Ethan-, n-Propan-, i-Propan-, n-Butan-, i-Butan-, sec.-Butan- und tert.-Butan-carbonsäurechlorid bzw. -bromid bzw. -anhydrid.

Die Verbindungen der Formel (III) sind bekannt.

Die als Ausgangsstoffe für das Verfahren (b) zu verwendenden Diaryl-ethylamino-Derivate sind durch die Formel (Ia) definiert. In dieser Formel stehen $R^2$, $R^3$, $R^4$, $R^5$ und $R^6$ vorzugsweise für diejenigen Reste, die bereits bei der Definition der Formel (I) genannt wurden.

Die Verbindungen der Formel (Ia) sind erfindungsgemäße Verbindungen. Sie lassen sich nach dem oben genannten Verfahren (a) auf einfacher Weise herstellen.

Die für die erfindungsgemäße Verfahren (b) ebenfalls als Ausgangsstoffe zu verwendenden Verbindungen sind durch die Formel (IV) definiert. In dieser Formel steht $R^1$ vorzugsweise für diejenigen Reste, die bereits bei der Definition der Formel (I) genannt wurden. X steht in dieser Formel für eine nucleophile Abgangsgruppe wie beispielsweise $-O-SO_2-OCH_3$, Chlorid, Bromid, Iodid oder Sulfat.

Die Verbindungen der Formel (IV) sind bekannten Verbindungen der organischen Chemie.

Das erfindungsgemäße Verfahren (a) zur Herstellung der neuen Diaryl-ethylamino-Derivate der Formel (I) kann in Gegenwart von Verdünnungsmitteln oder ohne Verdünnungsmittel durchgeführt werden.

Als Verdünnungsmittel kommen praktisch alle inerten Lösungsmittel in Frage. Hierzu gehören vorzugsweise aromatische Kohlenwasserstoffe, wie z.B. Benzol, Toluol und Xylol und Ether, wie z.B. Diethyl- und Diisopropylether, Tetrahydrofuran, Dioxan und 1,2-Dimethoxyethan.

Das Verfahren (a) kann gegebenenfalls in Gegenwart von Säureakzeptoren durchgeführt werden. Als Säureakzeptoren können alle üblichen Säurebindemittel Verwendung finden. Besonders bevorzugt sind Alkalicarbonat und -alkoholate, wie Natrium- und Kaliumcarbonat, Natrium- und Kaliummethylat bzw. -ethylat und -tert.-butylat, ferner aliphatische, aromatische oder heterocyclische Amine, beispielsweise Triethylamin, Trimethylamin, Dimethylanilin, Dimethylbenzylamin und Pyridin.

Bei der Verwendung von Säureanhydriden der Formel (III) als Ausgangsstoffe für das Verfahren (a) wird bevorzugt ohne Säureakzeptor gearbeitet. Als Verdünnungsmittel wird überschüssiges Säureanhydrid verwendet.

Bei der Verwendung von Säurehalogeniden der Formel (III) wird das erfindungsgemäße Verfahren (a) bevorzugt in Gegenwart von Verdünnungsmitteln und Säureakzeptoren durchgeführt.

Die Reaktionstemperatur kann beim erfindungsgemäßen Verfahren (a) zur Herstellung der neuen Verbindungen der Formel (I) innerhalb eines größeren Bereiches variiert werden. Im allgemeinen arbeitet man jeweils bei Temperaturen zwischen -20°C und +60°C, vorzugsweise zwischen 0°C und +40°C. Das erfindungsgemäße Verfahren (a) wird im allgemeinen unter Normaldruck durchgeführt.

Zur Durchführung des erfindungsgemäßen Verfahrens setzt man die Ausgangsstoffe (II) und (III) gewöhnlich in angenähert äquimolaren Mengen ein.

Bei der Verwendung von Säureanhydriden als Ausgangsstoffe der Formel (III) wird ein großer Überschuß an Säureanhydrid eingesetzt.

Die Komponenten werden gewöhnlich unter leichter Außenkühlung zusammengegeben, und das Reaktionsgemisch wird bis zum Reaktionsende gerührt. Die Aufarbeitung geschieht nach üblichen Methoden.

Bei der Durchführung des erfindungsgemäßen Verfahrens (b) können als Verdünnungsmittel vorzugsweise polare organische Lösungsmittel eingesetzt werden. Bevorzugt in Betracht kommen hierbei Ketone, wie z.B. Aceton, Methylethylketon, Methylisopropylketon und Methylisobutylketon, ferner Nitrile, außerdem Amide, wie Dimethylformamid, Dimethylacetamid und N-Methylpyrrolidon, sowie Ether, wie Diethyl- und Dibutylether, Glycoldimethylether und Diglycoldimethylether, Tetrahydrofuran und Dioxan.

Als Säureakzeptoren können bei der Durchführung des erfindungsgemäßen Verfahrens (b) vorzugsweise stark basische, jedoch schwach nucleophile Stoffe eingesetzt werden. Bevorzugt in Frage kommen Natriumhydrid, Kaliumhydrid, Calciumhydrid, Natriumcarbonat, Kaliumcarbonat

sowie Calciumcarbonat und Diazabicyclooctan (DABCO), Diazabicyclononen (DBN) und Diazabicycloundecen (DBU).

Die Reaktionstemperatur kann beim erfindungsgemäßen Verfahren (b) zur Herstellung der neuen Verbindungen der Formel (I) innerhalb eines größeren Bereiches variiert werden. Im allgemeinen arbeitet man bei Temperaturen zwischen 0°C und +100°C, vorzugsweise zwischen 0°C und 80°C. Das erfindungsgemäße Verfahren (b) wird im allgemeinen unter Normaldruck durchgeführt.

Zur Durchführung des erfindungsgemäßen Verfahrens (b) setzt man gewöhnlich die Ausgangsstoffe der Formel (Ia) und (IV) äquimolar ein oder verwendet einen leichten Überschuß an Verbindungen der Formel (IV).

Die Umsetzungen werden im allgemeinen in einem geeigneten Verdünnungsmittel und gegebenenfalls in Gegenwart eines Säureakzeptors durchgeführt. Die Aufarbeitung geschieht nach üblichen Methoden.

Die Wirkstoffe eignen sich bei guter Pflanzenverträglichkeit und günstiger Warmblütertoxizität zur Bekämpfung von tierischen Schädlingen, insbesondere Spinnentiere, die in der Landwirtschaft, in Forsten, im Vorrats- und Materialschutz sowie auf dem Hygienesektor vorkommen. Sie sind gegen normal sensible und resistente Arten sowie gegen alle oder einzelne

Entwicklungsstadien wirksam. Zu den oben erwähnten Schädlingen gehören:

Aus der Ordnung der Isopoda z.B. Oniscus asellus, Armadillidium vulgare, Porcellio scaber.
Aus der Ordnung der Diplopoda z.B. Blaniulus guttulatus.
Aus der Ordnung der Chilopoda z.B. Geophilus carpophagus, Scutigera spec.
Aus der Ordnung der Symphyla z.B. Scutigerella immaculata.
Aus der Ordnung der Thysanura z.B. Lepisma saccharina.
Aus der Ordnung der Collembola z.B. Onychiurus armatus.
Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.
Aus der Ordnung der Dermaptera z.B. Forficula auricularia.
Aus der Ordnung der Isoptera z.B. Reticulitermes spp..
Aus der Ordnung der Anoplura z.B. Phylloxera vastatrix, Pemphigus spp., Pediculus humanus corporis, Haematopinus spp., Linognathus spp.
Aus der Ordnung der Mallophaga z.B. Trichodectes spp., Damalinea spp.
Aus der Ordnung der Thysanoptera z.B. Hercinothrips femoralis, Thrips tabaci.

Aus der Ordnung der Heteroptera z.B. Eurygaster spp., Dysdercus intermedius, Piesma quadrata, Cimex lectularius, Rhodnius prolixus, Triatoma spp.

Aus der Ordnung der Homoptera z.B. Aleurodes brassicae, Bemisia tabaci, Trialeurodes vaporariorum, Aphis gossypii, Brevicoryne brassicae, Cryptomyzus ribis, Doralis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.

Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp., Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.

Aus der Ordnung der Hymenoptera z.B. Diprion spp., Hoplocampa spp., Lasius spp., Monomorium pharaonis, Vespa spp.

Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp..

Aus der Ordnung der Arachnida z.B. Scorpio maurus, Latrodectus mactans.

Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp..

Zu den pflanzenparasitären Nematoden gehören Pratylenchus spp., Radopholus similis, Ditylenchus dipsaci, Tylenchulus semipenetrans, Heterodera spp., Meloidogyne spp., Aphelenchoides spp., Longidorus spp., Xiphinema spp., Trichodorus spp..

Die erfindungsgemäßen Wirkstoffe weisen auch eine gute fungizide Wirkung gegen Pyricularia oryzae am Reis auf.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch

organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in ihren handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a.

Die erfindungsgemäßen Wirkstoffe können ferner in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepaßten üblichen Weise.

Bei der Anwendung gegen Hygiene- und Vorratsschädlinge zeichnen sich die Wirkstoffe durch eine hervorragende Residualwirkung auf Holz und Ton sowie durch eine gute Alkalistabilität auf gekälkten Unterlagen aus.

Beispiel A

Tetranychus-Test (resistent)

Lösungsmittel: 3 Gewichtsteile Dimethylformamid
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Bohnenpflanzen (Phaseolus vulgaris), die stark von allen Entwicklungsstadien der gemeinen Spinnmilbe oder Bohnenspinnmilbe (Tetranychus urticae) befallen sind, werden durch Tauchen in die Wirkstoffzubereitung der gewünschten Konzentration behandelt.

Nach der gewünschten Zeit wird die Abtötung in % bestimmt. Dabei bedeutet 100 %, daß alle Spinnmilben abgetötet wurden; 0 % bedeutet, daß keine Spinnmilben abgetötet wurden.

In diesem Test zeigen z.B. die folgenden Verbindungen der Herstellungsbeispiele eine bessere akarizide Wirksamkeit als die Vergleichssubstanz (A):
(1), (3), (14), (15), (22), (26), (31), (34), (35), (37) und (40).

Herstellungsbeispiele

Beispiel 1

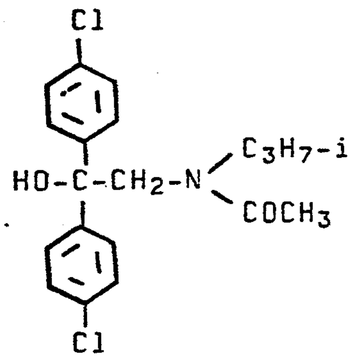

(Verfahren (a))

Eine Lösung von 2,36 g (0,03 Mol) Acetylchlorid in 20 ml Dioxan wurde unter Rühren zu einer mit Eis gekühlten Mischung aus 9,7 g (0,03 Mol) /2,2-Bis-(4-chlorphenyl)-2-hydroxyethyl7-(1-methylethyl)-amin und 3,16 g (0,04 Mol) Pyridin in 60 ml Dioxan tropfenweise gegeben.

Das Reaktionsgemisch wurde 15 Stunden bei 20°C gerührt, anschließend vom Feststoff abfiltriert und die Lösung unter vermindertem Druck eingeengt. Der Rückstand wurde in Essigsäureethylester und Wasser aufgenommen, die organische Phase abgetrennt und über Natriumsulfat getrocknet. Das Lösungsmittel wurde unter vermindertem Druck entfernt.

Man erhielt 10,1 g (92 % der Theorie) N-/2,2-Bis-(4-chlorphenyl)-2-hydroxyethyl/-N-(1-methylethyl)-acetamid vom Schmelzpunkt 98°C.

Beispiel 2

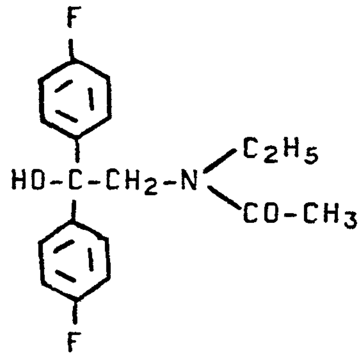

(Verfahren (a))

5,54 g (0,02 Mol) /2,2-Bis-(4-fluorphenyl)-2-hydroxyethyl/-ethylamin wurden in 40 ml Essigsäureanhydrid eingetragen und 15 Stunden bei 20°C gerührt. Das Reaktionsgemisch wurde in 200 ml Eiswasser eingegossen, die Mischung 2 Stunden gerührt und der ausgefallene Niederschlag anschließend abgenutscht, mit Wasser gewaschen und im Vakuum getrocknet.

Man erhielt 5,89 g (92,3 % der Theorie) N-/2,2-Bis-(4-fluorphenyl)-2-hydroxyethyl/-N-ethylacetamid vom Schmelzpunkt 128°C.

0167019

Beispiel 3

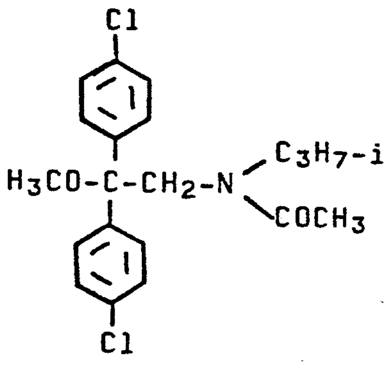

(Verfahren (b))

Zu einer Lösung von 3,66 g (0,01 Mol) N-/2,2-Bis-(4-chlorphenyl)-2-hydroxyethyl/-N-(1-methylethyl)-acetamid in 50 ml Tetrahydrofuran und 25 ml Dimethylformamid wurden bei 20°C in kleinen Portionen 0,45 g (0,015 Mol) Natriumhydrid (80 %ig in Paraffin) gegeben und die Reaktionsmischung wurde bis zum Ende der Wasserstoffentwicklung auf 40°C erwärmt. Anschließend wurden bei 40°C 2,13 g (0,015 Mol) Methyliodid zugetropft und das Reaktionsgemisch wurde 30 Minuten bei 40°C und 15 Stunden bei 20°C gerührt. Der Niederschlag wurde abfiltriert und mit Tetrahydrofuran nachgewaschen. Das Filtrat und die Waschlösung wurden eingeengt und der Rückstand in Dichlormethan und Wasser aufgenommen. Die organische Phase wurde abgetrennt, über Natriumsulfat getrocknet und im Vakuum eingeengt.

Man erhielt 3,6 g (94,7 % der Theorie) N-[2,2-Bis-(4-chlorphenyl)-2-methoxyethyl]-N-(1-methylethyl)-acetamid.

$^{1}$H-NMR($CDCl_3$) : δ = 3,94 - 4,28 (d, 2H, $-\overset{|}{\underset{|}{C}}-CH_2-\overset{|}{N}-$) ppm.

Analog den Beispielen 1, 2 und 3 bzw. Verfahren (a) und (b) wurden die folgenden Verbindungen der Formel (I) hergestellt:

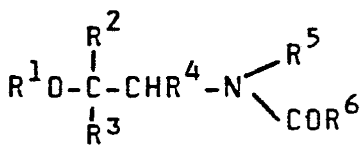

(I)

**Tabelle 2**

| Beisp. Nr. | $R^1$ | Verfahren | $R^2$ | $R^3$ | $R^4$ | $R^5$ | $R^6$ | Phys.Konstante Fp bzw. $^1H$-NMR($CDCl_3$)/ [ppm] |
|---|---|---|---|---|---|---|---|---|
| 4 | H | (a) | Cl-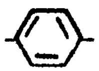 | Cl-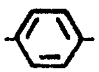 | H | i-$C_4H_9$ | $CH_3$ | $\delta$ = 4,17 (s, 2H, $-CH_2-$) |
| 5 | H | (a) | Cl-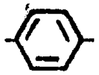 | Cl-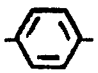 | H | i-$C_3H_7$ | i-$C_4H_9$ | $\delta$ = 4,02 (s, 2H, $-CH_2-$) |
| 6 | H | (a) | Cl-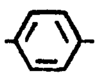 | Cl-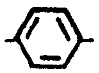 | H | i-$C_3H_7$ | $C_2H_5$ | 102 °C |
| 7 | H | (a) | Cl-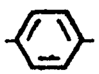 | Cl-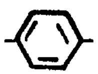 | H | i-$C_3H_7$ | i-$C_3H_7$ | 100 °C |
| 8 | H | (a) | Cl-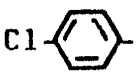 | Cl-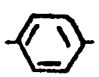 | H | i-$C_3H_7$ | H | 176 °C |
| 9 | H | (a) | Cl-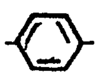 | Cl-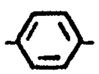 | H | 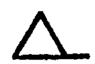 | $CH_3$ | 131 °C |
| 10 | H | (a) | Cl-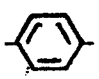 | Cl-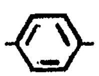 | H | i-$C_3H_7$ | $CH_3$ | 102 °C |
| 11 | H | (a) | Cl-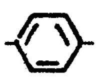 | $H_3CO$-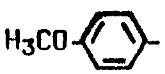 | H | i-$C_3H_7$ | $CH_3$ | $\delta$ = 4,00 (s, 2H, $-CH_2-$) |
| 12 | H | (a) | Cl-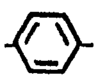 | Cl-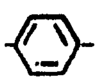 | H | n-$C_4H_9$ | $CH_3$ | 80 °C |
| 13 | H | (a) | Cl-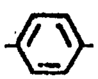 | Cl-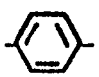 | H | sec.-$C_4H_9$ | $CH_3$ | 108 °C |
| 14 | H | (a) | Cl-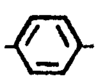 | Cl-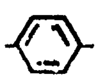 | H | $CH_3$ | $CH_3$ | 115 °C |

Tabelle 2 - Fortsetzung

| Beisp. Nr. | $R^1$ | Verfahren | $R^2$ | $R^3$ | $R^4$ | $R^5$ | $R^6$ | Phys.Konstante Fp bzw. $^1H$-NMR($CDCl_3$)/ [ppm] |
|---|---|---|---|---|---|---|---|---|
| 15 | H | (a) | 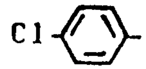 | 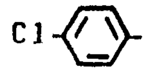 | H | $C_2H_5$ | $CH_3$ | 136 °C |
| 16 | H | (a) | 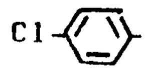 | 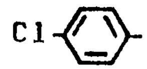 | H | 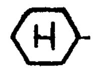 | $CH_3$ | 125 °C |
| 17 | H | (a) | 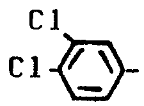 | 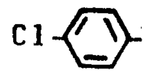 | H | $i-C_3H_7$ | $CH_3$ | 102 - 104 °C |
| 18 | H | (a) | 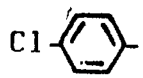 | 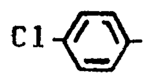 | H | 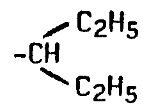 | $CH_3$ | 124 °C |
| 19 | H | (a) | 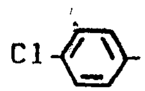 | 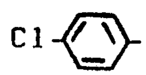 | H | 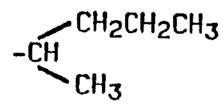 | $CH_3$ | $\delta$ = 4,08 (s, 2H, $-CH_2-$) |
| 20 | H | (a) | 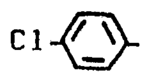 | 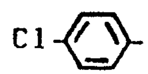 | H | 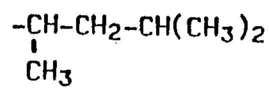 | $CH_3$ | $\delta$ = 4,08 (s, 2H, $-CH_2-$) |
| 21 | H | (a) | 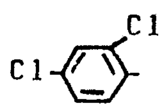 | 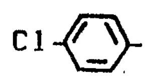 | H | $i-C_3H_7$ | $CH_3$ | $\delta$ = 4,10 - 4,78 (m, 2H, $-CH_2-$) |
| 22 | H | (a) | 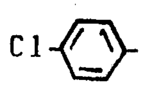 | 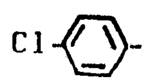 | H | $-CH_2-CH=CH_2$ | $CH_3$ | 104 - 106 °C |

Tabelle 2 - Fortsetzung

| Beisp. Nr. | $R^1$ | Verfahren | $R^2$ | $R^3$ | $R^4$ | $R^5$ | $R^6$ | Phys.Konstante Fp bzw. $^1H$-NMR($CDCl_3$)/ [ppm] |
|---|---|---|---|---|---|---|---|---|
| 23 | H | (a) | 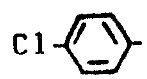 | 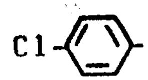 | H | $-C_3H_7-n$ | $CH_3$ | 110 °C |
| 24 | H | (a) | 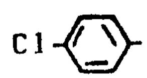 | 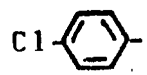 | H | $-CH_2-CH(OH)-CH_3$ | $CH_3$ | $\delta$ = 4,03 - 4,54 (m, 2H, $-CH_2-$) |
| 25 | H | (a) | 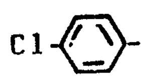 | 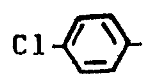 | H | $-CH_2-CH_2OCH_3$ | $CH_3$ | $\delta$ = 4,23 (s, 2H, $-CH_2-$) |
| 26 | H | (a) | 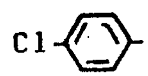 | 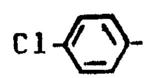 | H | $-CH_2-C\equiv CH$ | $CH_3$ | 154 °C |
| 27 | H | (a) | 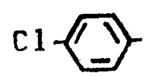 | 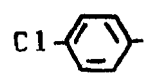 | H | $C_2H_5$ | $C_2H_5$ | 82 °C |
| 28 | H | (a) | 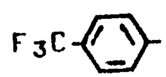 | 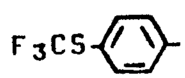 | H | $i-C_3H_7$ | $CH_3$ | $\delta$ = 4,17 (s, 2H, $-CH_2-$) |
| 29 | H | (a) | 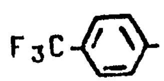 | 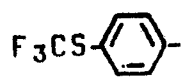 | H | $C_2H_5$ | $CH_3$ | 83 °C |
| 30 | H | (a) | 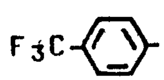 | 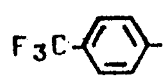 | H | $C_2H_5$ | $CH_3$ | 138 °C |
| 31 | H | (a) | 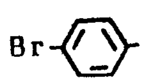 | 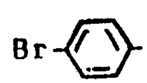 | H | $C_2H_5$ | $CH_3$ | 107 °C |
| 32 | H | (a) | 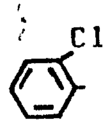 | 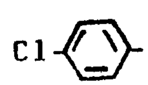 | H | $i-C_3H_7$ | $CH_3$ | 104 °C |

Tabelle 2 - Fortsetzung

| Beisp. Nr. | $R^1$ | Verfahren | $R^2$ | $R^3$ | $R^4$ | $R^5$ | $R^6$ | Phys.Konstante Fp bzw. $^1H$-NMR($CDCl_3$)/ [ppm] |
|---|---|---|---|---|---|---|---|---|
| 33 | H | (a) | 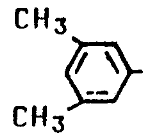 | 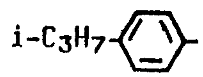 | H | $i-C_3H_7$ | $CH_3$ | $\delta$= 4,10 (s, 2H, $-CH_2-$) |
| 34 | $C_2H_5$ | (b) | 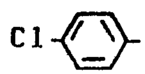 | 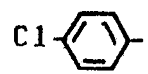 | H | $i-C_3H_7$ | $CH_3$ | $\delta$= 4,16 (s, 2H, $-CH_2-$) |
| 35 | 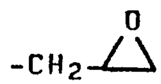 | (b) | 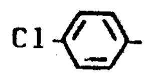 | 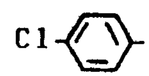 | H | $i-C_3H_7$ | $CH_3$ | $\delta$= 4,05 (s, 2H, $-CH_2-$) |
| 36 | $-CH_2-C\equiv CH$ | (b) | 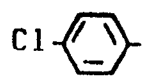 | 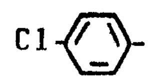 | H | $i-C_3H_7$ | $CH_3$ | $\delta$= 4,25 (s, 2H, $-CH_2-$) |
| 37 | $-CH_2-CH=CH_2$ | (b) | 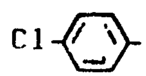 | 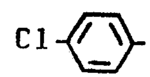 | H | $i-C_3H_7$ | $CH_3$ | $\delta$= 4,20 (s, 2H, $-CH_2-$) |
| 38 | H | (a) | 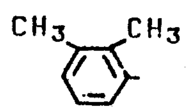 | 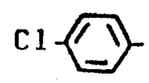 | H | $i-C_3H_7$ | $CH_3$ | $\delta$= 3,67 - 4,58 (m, 2H, $-CH_2-$) |
| 39 | H | (a) | 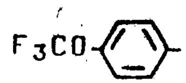 | 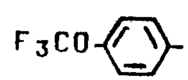 | H | $i-C_3H_7$ | $CH_3$ | 60 °C |
| 40 | H | (a) | 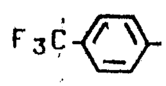 | 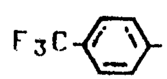 | H | $i-C_3H_7$ | $CH_3$ | 100 °C |

- Fortsetzung

| Beisp. Nr. | $R^1$ | Verfahren | $R^2$ | $R^3$ | $R^4$ | $R^5$ | $R^6$ | Phys.Konstante Fp bzw. $^1H$-NMR($CDCl_3$)/ [ppm] |
|---|---|---|---|---|---|---|---|---|
| 41 | H | (a) | $F_3C$- 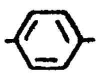 | $F_3CO$- 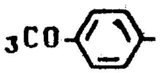 | H | i-$C_3H_7$ | $CH_3$ | 78 °C |
| 42 | H | (a) | Cl- 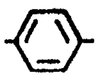 | Cl- 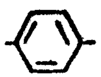 | $CH_3$ | $C_2H_5$ | $CH_3$ | 168 °C |
| 43 | H | (a) | $F_3C$- 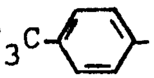 | $F_3C$- 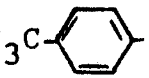 | H | $-CH_2-CH=CH_2$ | $CH_3$ | 72°C |
| 44 | H | (a) | $F_3C$- 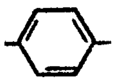 | $F_3C$- 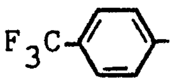 | H | $CH_3$ | $CH_3$ | 88 - 90°C |
| 45 | H | (a) | F- 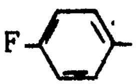 | F- 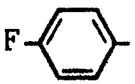 | H | i-$C_3H_7$ | $CH_3$ | 102°C |
| 46 | $CH_3$ | (b) | Cl- 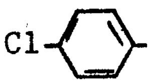 | Cl- 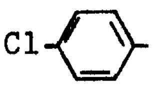 | H | $C_2H_5$ | $CH_3$ | $\delta$= 4,31 (s, 2H,$-CH_2-$) |
| 47 | H | (a) | F- 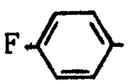 | F- 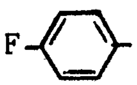 | H | $-CH_2-C\equiv CH$ | $CH_3$ | 101°C |
| 48 | H | (a) | F- 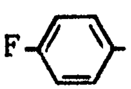 | F- 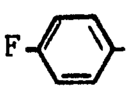 | H | $-CH_3$ | $CH_3$ | 116°C |
| 49 | H | (a) | Br- 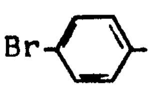 | Br- 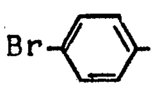 | H | $-CH_2-CH=CH_2$ | $CH_3$ | 137°C |
| 50 | H | (a) | Br- 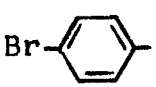 | Br- 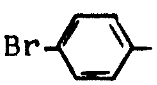 | H | i-$C_3H_7$ | $CH_3$ | $\delta$= 4,07 (s, 2H,$-CH_2-$) |
| 51 | H | (a) | I- 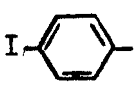 | I- 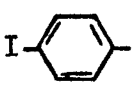 | H | i-$C_3H_7$ | $CH_3$ | 128 - 13°C |
| | | (a) | Br- 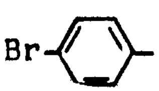 | Cl- 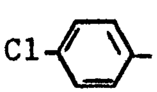 | H | $C_2H_5$ | $CH_3$ | $\delta$= 4 [illegible] |

Tabelle 2 - Fortsetzung

| Beisp. Nr. | $R^1$ | Verfahren | $R^2$ | $R^3$ | $R^4$ | $R^5$ | $R^6$ | Phys. Konstante Fp bzw $^1H$-NMR($CDCI_3$)/ [ppm] |
|---|---|---|---|---|---|---|---|---|
| 53 | H | (a) | 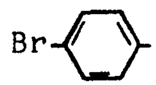 | 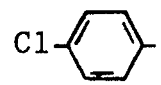 | H | i-$C_3H_7$ | $CH_3$ | $\delta$ = 4,02 (s, 2H, -CH-) |
| 54 | H | (a) | 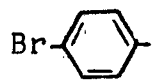 | 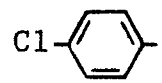 | H | -Cl-C≡CH | $CH_3$ | $\delta$ = 4,22 (s, 2H, $-CH_2-$) |
| 55 | H | (a) | 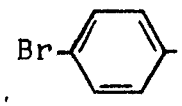 | 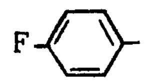 | H | i-$C_3H_7$ | $CH_3$ | 146°C |
| 56 | H | (a) | 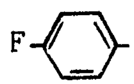 | 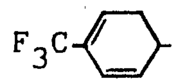 | H | i-$C_3H_7$ | $CH_3$ | 115°C |
| 57 | H | (a) | 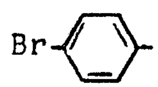 | 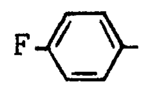 | H | $C_2H_5$ | $CH_3$ | 96°C |

0167019

Patentansprüche

1. Diaryl-ethylamino-Derivate der Formel

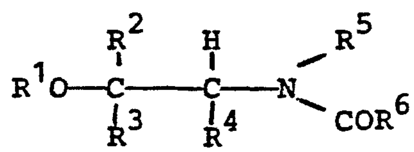

(I)

in welcher

$R^1$ für Wasserstoff, gegebenenfalls substituiertes Alkyl, Alkenyl oder Alkinyl steht,

$R^2$ und $R^3$ gleich oder verschieden sind und für substituiertes Aryl stehen,

$R^4$ für Wasserstoff oder Alkyl steht,

$R^5$ für gegebenenfalls substituiertes Alkyl, für Alkenyl, Alkinyl oder Cycloalkyl steht und

$R^6$ für Wasserstoff oder Alkyl steht.

2. Diaryl-ethylamino-Derivate der Formel (I) gemäß Anspruch 1 in welcher

$R^1$ für Wasserstoff oder für gegebenenfalls substituiertes Alkyl mit 1 bis 6 Kohlenstoffatomen, für Alkenyl oder Alkinyl mit 2 bis 6 Kohlenstoffatomen steht,

$R^2$ und $R^3$ gleich oder verschieden sind und für einfach oder mehrfach, gleich oder verschieden substituiertes Aryl mit 6 bis 10 Kohlenstoffatomen stehen,

$R^4$ für Wasserstoff oder Alkyl mit 1 bis 4 Kohlenstoffatomen steht,

$R^5$ für gegebenenfalls substituiertes Alkyl mit 1 bis 6 Kohlenstoffatomen, Alkenyl und Alkinyl mit jeweils 2 bis 6 Kohlenstoffatomen sowie für Cycloalkyl mit 3 bis 7 Kohlenstoffatomen steht,

und

$R^6$ für Wasserstoff oder Alkyl mit 1 bis 6 Kohlenstoffatomen steht.

3. Verfahren zur Herstellung von Diaryl-ethylamino-Derivate der Formel

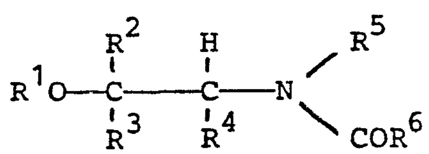

(I)

in welcher

$R^1$ für Wasserstoff, gegebenenfalls substituiertes Alkyl, Alkenyl oder Alkinyl steht,

$R^2$ und $R^3$ gleich oder verschieden sind und für substituiertes Aryl stehen,

$R^4$ für Wasserstoff oder Alkyl steht,

$R^5$ für gegebenenfalls substituiertes Alkyl, für Alkenyl, Alkinyl oder Cycloalkyl steht

und

$R^6$ für Wasserstoff oder Alkyl steht,

dadurch gekennzeichnet, daß man

(a) Amine der Formel (II)

$$R^1O\text{-}CR^2R^3\text{-}CHR^4\text{-}NHR^5 \qquad (II)$$

in welcher

$R^1$, $R^2$, $R^3$, $R^4$ und $R^5$ die oben angegebenen Bedeutungen haben,

mit Verbindungen der Formel (III)

$$R^6\text{-}\overset{\overset{\displaystyle O}{\|}}{C}\text{-}Y \qquad (III)$$

in welcher

$R^6$ die oben angegebene Bedeutung hat und

Y für Chlor, Brom oder für den Rest der Formel $-O-\overset{O}{\overset{\|}{C}}-R^7$ steht,

in welcher

$R^7$ für Alkyl steht,

gegebenenfalls in Gegenwart von Verdünnungsmitteln und gegebenenfalls in Gegenwart von Säureakzeptoren umsetzt,

oder

(b) Diaryl-ethylamino-Derivate der Formel (Ia)

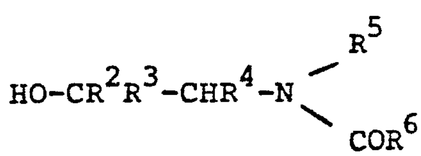

(Ia)

in welcher

$R^2$, $R^3$, $R^4$, $R^5$ und $R^6$ die oben angegebenen Bedeutungen haben,

mit Alkylierungsmitteln der Formel (IV)

$$R^1X \qquad (IV)$$

in welcher

$R^1$ die oben angegebene Bedeutung hat und

X für eine nucleophile Abgangsgruppe steht,

in Gegenwart von Säureakzeptoren und gegebenenfalls in Gegenwart von Verdünnungsmitteln umsetzt.

4. Schädlingsbekämpfungsmittel, gekennzeichnet durch einen Gehalt an mindestens einer Diaryl-ethylamino-Verbindung der Formel (I).

5. Verfahren zur Bekämpfung von tierischen Schädlingen und von Pilzen, dadurch gekennzeichnet, daß man Diaryl-ethylamino-Derivate der Formel (I) auf tierische Schädlinge und/oder Pilzen oder auf den Lebensraum tierischer Schädlinge und/oder Pilze einwirken läßt.

6. Verwendung von Diaryl-ethylamino-Verbindungen der Formel (I) zur Bekämpfung von tierischen Schädlingen und/oder Pilzen.

7. Verfahren zur Herstellung von Schädlingsbekämpfungsmitteln, dadurch gekennzeichnet, daß man Diaryl-ethylamino-Verbindungen der Formel (I) mit Streckmitteln und/oder oberflächenaktiven Mitteln vermischt.

0167019

Europäisches Patentamt

# EUROPÄISCHER RECHERCHENBERICHT

Nummer der Anmeldung

EP 85 10 7107

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl 4) |
|---|---|---|---|
| X | EP-A-0 075 868 (SUMITOMO CHEMICAL CO. LTD.) * Seiten 3,9,10; Ansprüche 1,3 * | 1,3 | C 07 C 103/38<br>C 07 C 103/60<br>A 01 N 37/18 |
| A | BULLETIN OF THE CHEMICAL SOCIETY OF JAPAN, Band 53, Nr. 4, April 1980, Seiten 1183-1184, Yamaguchi University, Yoshida, JP; T. TOKUMITSU et al.: "Reactions of enamino kotones. VII. The photochemical reaction of 4-methylamino-3-penten-2-one and 2,4-pentanedione with benzophenone" * Seite 1183, rechter Spalte, Zeilen 1-2 * | 1 | |
| | | | RECHERCHIERTE SACHGEBIETE (Int. Cl.4) |
| | | | C 07 C 103/00<br>A 01 N 37/00 |

Der vorliegende Recherchenbericht wurde für alle Patentansprüche erstellt.

| Recherchenort | Abschlußdatum der Recherche | Prüfer |
|---|---|---|
| DEN HAAG | 25-09-1985 | MOREAU J.M. |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument